# EUROPEAN PATENT SPECIFICATION

(11) **EP 4 360 659 B1**
(45) Date of publication and mention of the grant of the patent: **30.04.2025**
(21) Application number: 23203605.3
(22) Date of filing: 13.10.2023
(51) Int. Cl.: A61K 49/00

(54) **FLUORESCENT WATER-SOLUBLE POLYCATIONIC CHITOSAN POLYMERS AS MARKERS FOR BIOLOGICAL 3D IMAGING**
FLUORESZIERENDE WASSERLÖSLICHE POLYKATIONISCHE CHITOSANPOLYMERE ALS MARKER FÜR BIOLOGISCHE 3D-BILDGEBUNG
POLYMÈRES FLUORESCENTS DE CHITOSANE POLYCATIONIQUE HYDROSOLUBLES UTILISÉS COMME MARQUEURS POUR L'IMAGERIE 3D BIOLOGIQUE

(30) Priority: 26.10.2022 IT 202200022113
(43) Date of publication of application: 01.05.2024
(73) Proprietor: Cyanagen S.r.l., 40138 Bologna (IT)
(72) Inventor: PERCIACCANTE, Rossana, I-40138 Bologna (IT); VAJPAYEE, Srishti, I-40138 Bologna (IT); JANSEN, Thomas Paul, I-40138 Bologna (IT); PICASCIA, Tiziana, I-40138 Bologna (IT); GRETZ, Norbert, deceased (DE)
(74) Representative: Buzzi, Notaro & Antonielli d'Oulx S.p.A.

(56) References cited:
- WO-A1-2009/152440
- WO-A1-2015/066290
- AU-A1- 2010 236 189
- CA-A1- 3 044 891
- THOMAS REJU GEORGE ET AL: "MHI-148 Cyanine Dye Conjugated Chitosan Nanomicelle with NIR Light-Trigger Release Property as Cancer Targeting Theranostic Agent", MOLECULAR IMAGING & BIOLOGY, ELSEVIER, BOSTON, vol. 20, no. 4, 15 February 2018 (2018-02-15), pages 533 - 543, XP036545004, ISSN: 1536-1632, [retrieved on 20180215], DOI: 10.1007/S11307-018-1169-Z
- SRINIVASAN SUPRIYA ET AL: "Near-infrared fluorescing IR820-chitosan conjugate for multifunctional cancer theranostic applications", JOURNAL OF PHOTOCHEMISTRY AND PHOTOBIOLOGY B: BIOLOGY, ELSEVIER SCIENCE S.A., BASEL, CH, vol. 119, 28 December 2012 (2012-12-28), pages 52 - 59, XP028970491, ISSN: 1011-1344, DOI: 10.1016/J.JPHOTOBIOL.2012.12.008
- MARC VENDRELL ET AL: "Synthesis and characterization of a cell-permeable near-infrared fluorescent deoxyglucose analogue for cancer cell imaging", ORGANIC & BIOMOLECULAR CHEMISTRY, vol. 9, no. 13, 12 May 2011 (2011-05-12), pages 4760 - 4762, XP055770835, ISSN: 1477-0520, DOI: 10.1039/c1ob05519d
- LI XUDONG ET AL: "Chemical Modification of Chitosan for Developing Cancer Nanotheranostics", BIOMACROMOLECULES, vol. 23, no. 6, 13 June 2022 (2022-06-13), US, pages 2197 - 2218, XP093041491, ISSN: 1525-7797, DOI: 10.1021/acs.biomac.2c00184
- SASHIWA HITOSHI ET AL: "Chemical Modification of Chitosan. 14: Synthesis of Water-Soluble Chitosan Derivatives by Simple Acetylation", BIOMACROMOLECULES, vol. 3, no. 5, 18 July 2002 (2002-07-18), US, pages 1126 - 1128, XP093042079, ISSN: 1525-7797, DOI: 10.1021/bm0200480
- HU XIAO-XIAO ET AL: "A near infrared colorimetric and fluorometric probe for organophosphorus nerve agent mimics by intramolecular amidation", CHEMICAL COMMUNICATIONS, vol. 51, no. 82, 20 August 2015 (2015-08-20), UK, pages 15118 - 15121, XP093042068, ISSN: 1359-7345, DOI: 10.1039/C5CC04630K

## Description

The project leading to this application has received funding from the European Union's Horizon 2020 research and innovation programme under the Marie Sklodowska-Curie grant agreement No 813839.

### FIELD OF INVENTION

The present invention relates to fluorescent water-soluble polycationic chitosan polymers and use thereof as imaging agents for imaging glycosamine-containing structures, e.g., vascular structures, 3-dimensionally.

### BACKGROUND OF THE INVENTION

Vascular studies are important to understand the structure and function of arteries, capillaries, and veins. This information is useful to understand if there is a proper blood flow through different organs. Compromised vascular functioning is associated with many diseases, such as atherosclerosis, aneurysm, hypertension, chronic kidney diseases, etc. [1].

This task has been approached by various methods. Ultrasound (US) is mostly used in clinics. It can measure the amount of blood pumped with each heartbeat, which indicates the measure of vascular diameter. US can also detect abnormal blood flow, indicating blockage. However, this technique has very low spatial resolution, is sensitive towards artifacts (such as movement artifacts) and has a high cost of instrumentation [1,2].

Magnetic resonance imaging (MRI) is used to study vascular anatomy and blood flow to learn more about disease progression or is part of routine check-up. But it uses expensive instrumentation, along with expensive contrast agents. MRI provides low spatial resolution and sensitivity and is prone to artifacts, in addition its use is limited in patients with pacemakers or metallic implants [1,2].

Like MRI, computed tomography (CT) can be used to study blood vessels. It is used to diagnose acute coronary symptoms and aneurysms. CT has a higher spatial resolution than US and MRI. But it has serious problems with the use of ionising radiation and the expensive contrast agents. Furthermore, the contrast media can be nephrotoxic [1,2].

A substantial improvement is the use of optical imaging with fluorescence-based staining of intra- and extra-cellular biomolecules. Fluorescent markers such as Alcian Blue, indocyanine green dye, green fluorescent proteins and various synthetic chromophores have been used for the vascular visualisation [1]. Vascular biopsies combined with cellular staining is a common procedure in hospitals for diagnostic purposes. It is very efficient in site-specific study of tissue abnormalities, but is invasive, and can lead to infection, damage to nearby tissues, or excessive bleeding. Fluorescently labelled antibodies are another example for using fluorescence based vascular visualisation. For example, Alexa Fluor 647 labelled anti-CD31 is used for staining the endothelial cell lining of the vessels. This technique offers high specificity for the target but is very expensive. Moreover, the wavelength of these dyes is mainly in the blue and green region of the spectrum. This fact limits their application in visualising and imaging the inner organ structures because of the poor tissue penetration depth and the disturbance of the signal by autofluorescence of the tissue, compared to the red region of the spectrum [1].

Recently, many studies have focused on the use of fluorescent polymers instead of antibodies as a cheaper alternative to a diverse range of therapeutic and diagnostic biomedical applications, such as tools for gene delivery, immunotherapy, visualisation of tissue structures etc.

WO 2018/100089 demonstrates the use of one such fluorescent polymer for vascular staining. It describes the application of polyethyleneimine (PEI) conjugated to MHI148 dye for the visualisation and counting of glomeruli. MHI148-PEI uses branched PEI of molecular weight 70 kDa, conjugated to a cationic cyanine 7 dye and binds to glycosamine containing biological structures basing on an electrostatic interaction [3].

However, this compound has multiple drawbacks. MHI148 dye is well known for its chemical instability. It consists of a meso-positioned amino alkyl chain, which is sensitive to chemical and photoinduced decomposition [4]. Also, the use of PEI is limited in biological settings because of its toxicity. PEI has been reported to induce cellular toxicity and hence creates problems with the Regulatory Affairs [5].

Thomas, R.G., et al, (2018) discloses cancer-targeting heptamethine dye MHI-148 conjugated to a hydrophobically modified (5β-cholanic acid) glycol chitosan (forming nanomicelle).

There is therefore the need of new fluorescent markers for use in visualising glycosamine-containing structures, like blood vessels, that are chemically stable, non-toxic, and more efficient in imaging.

### OBJECTIVE AND SUMMARY OF THE INVENTION

The object of the present invention is to provide a novel substance which can be used as an imaging agent to study vascular structures and that overcomes the disadvantages of the markers known in the prior art.

An object of the present invention is to provide new fluorescent markers that are chemically stable.

A further object of the present invention is to provide fluorescent markers that are non-toxic for their use in *in vivo* or *ex vivo* imaging of glycosamine-containing structures.

A further object of the present invention is to provide fluorescent markers that are more efficient in visualising glycosamine-containing structures than the known markers.

A further object of the present invention is to provide fluorescent markers that deliver reproducible results in visualising glycosamine-containing structures.

According to the invention, the above objects are achieved thanks to the matter specified in the ensuing claims, which are understood as forming an integral part of the present invention.

The current invention concerns new fluorescent polymers as a non-toxic, cheap, reproducible, and more efficient alternative than the known markers for imaging glycosamine-containing structures using 3-dimensional imaging.

An embodiment of the present invention relates to a fluorescent water-soluble polycationic chitosan polymer of formula (I) and salts thereof: 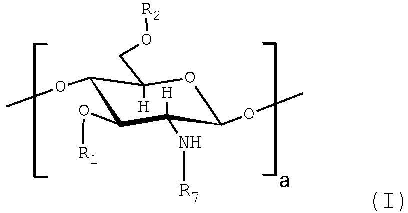 wherein
a is an integer between 220 and 11,200;
R₁ and R₂ are independently selected from the group consisting of CH₃CO and H, provided that at least 25% of the *O*-positions of the chitosan polymer structure is substituted with CH₃CO;
R₇ is selected from the group consisting of CH₃CO, H and Dye, provided that (i) at least one Dye residue is present in the chitosan polymer structure, and (ii) at least 15% of the *N*-positions of the chitosan polymer structure is substituted with CH₃CO;
Dye is a polymethine dye, having a general formula (II) : 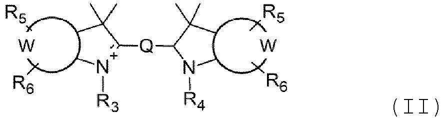
wherein
W represents 4-8 carbon atoms forming a benzocondensed or a naphto-condensed ring;
R₃ and R₄ are independently selected from the group consisting of CH₂CH₃, (CH₂)₃SO³⁻, and (CH₂)₃N⁺(CH₃)₃ and salts thereof;
R₅ and R₆ are independently selected from the group consisting of H, and SO³⁻ and salts thereof; and
Q is selected from the group consisting of 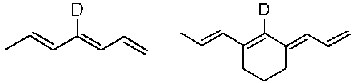
wherein D is selected from the group consisting of

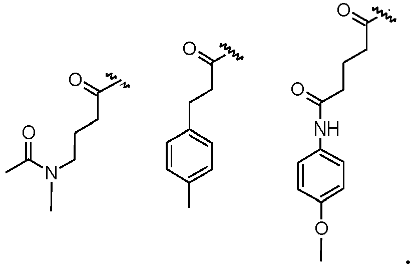

A further embodiment of the present invention relates to an *in vitro* or *ex vivo* method of imaging a glycosamine-containing structure in a biological sample using a fluorescent water-soluble polycationic chitosan polymer of formula (I).

A further embodiment of the present invention relates to a fluorescent water-soluble polycationic chitosan polymer of formula (I) for use in an *in vivo* imaging method of a glycosamine-containing structure in a mammal.

The current invention concerns also a kit comprising at least one fluorescent water-soluble polycationic chitosan polymer of formula (I) for the application in a 3D imaging method of a glycosamine-containing structure.

### BRIEF DESCRIPTION OF THE DRAWINGS

The invention will now be described in detail, purely by way of illustrative and non-limiting example, with reference to the attached figures, wherein:
**Figure 1****.** Chemical structures of fluorescent water-soluble polycationic chitosan polymers where WSC stands for water-soluble chitosan.

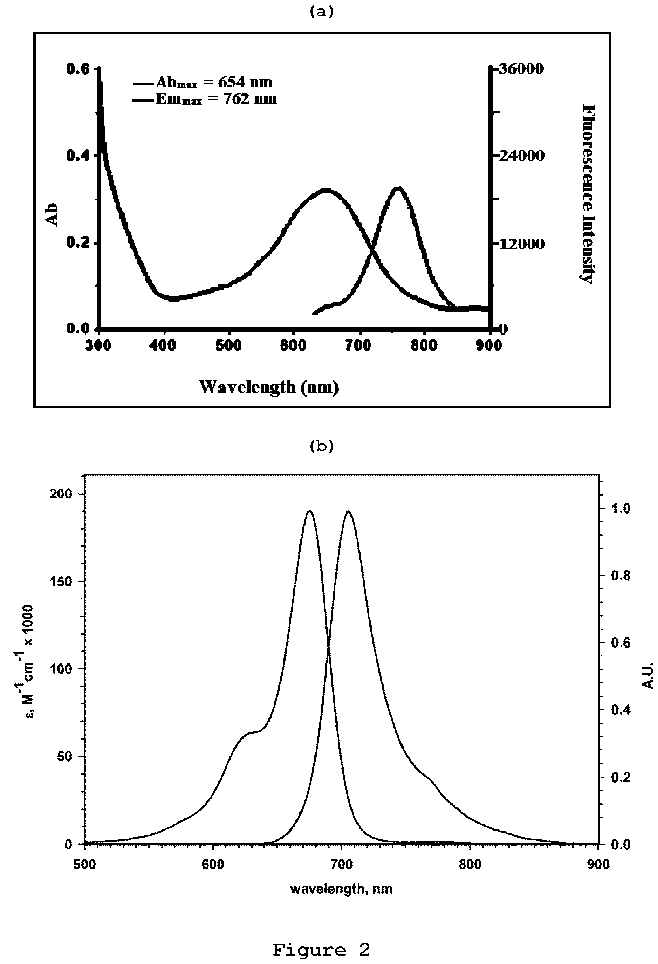

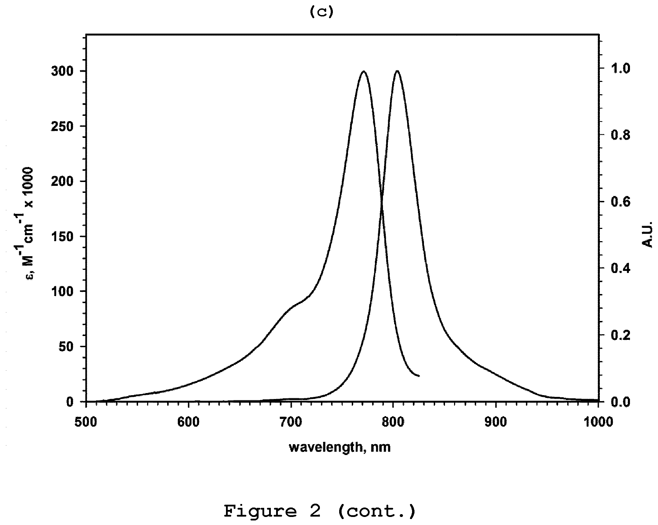

**Figure 2****.** Electromagnetic spectrum of fluorescent water-soluble polycationic chitosan polymers:
   (a) SV620C-01-WS chitosan,
   (b) SV680A-02-WS chitosan and SV680A-03-WS chitosan,
   (c) SV770Z-01-WS chitosan, SV770C-01-WS chitosan and SV770C-02-WS chitosan.
**Figure 3****.** Photostability studies performed on fluorescent polycationic polymers:
   (a) SV620C-01-PEI,
   (b) SV620C-01-WS chitosan,
   (c) SV680A-02-WS chitosan,
   (d) SV680A-03-WS chitosan,
   (e) SV770Z-01-WS chitosan.
**Figure 4****.** Renal vascular network from a mouse kidney pole. Imaged by Confocal microscopy, excitation at 770 nm, using a 16x objective in ECi.
**Figure 5****.** Renal vascular network from a mouse kidney pole. Imaged by Confocal microscopy, excitation at 638 nm, using a 16x objective in immersion oil.
**Figure 6****.** Renal vascular network from mouse kidney sections (cortical area) in details. From left to right: (1) SV770Z-01-WS Chitosan staining, (2) SV680A-02-WS Chitosan staining, (3) MHI148-PEI staining.

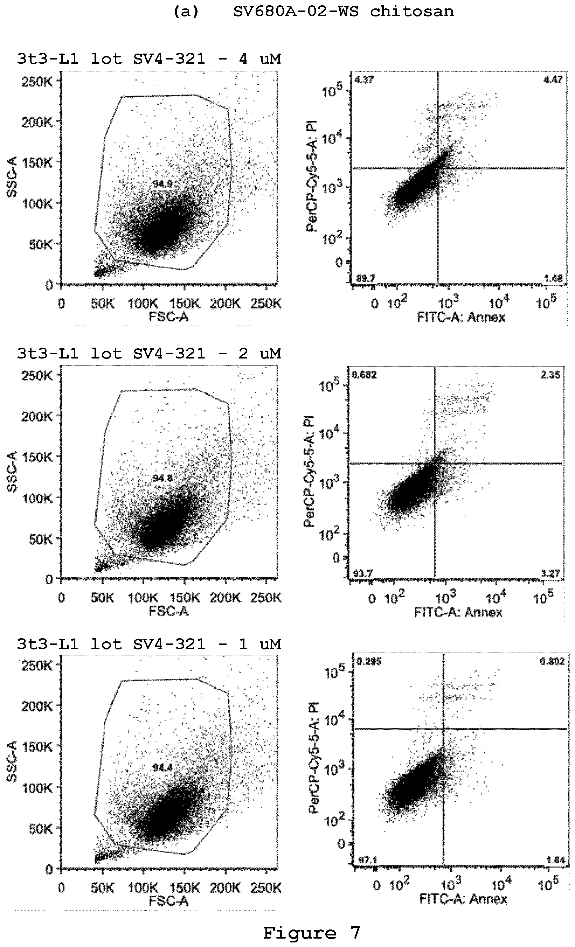

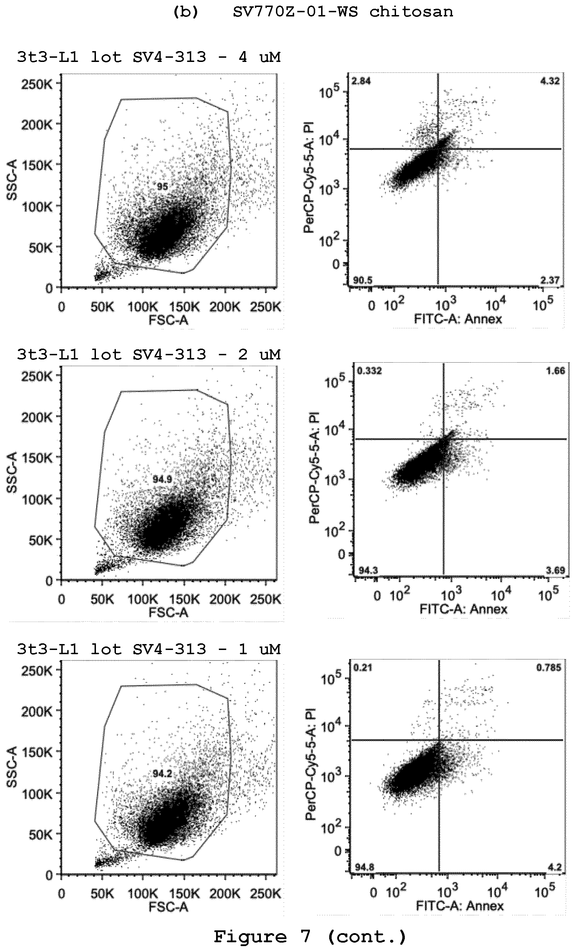

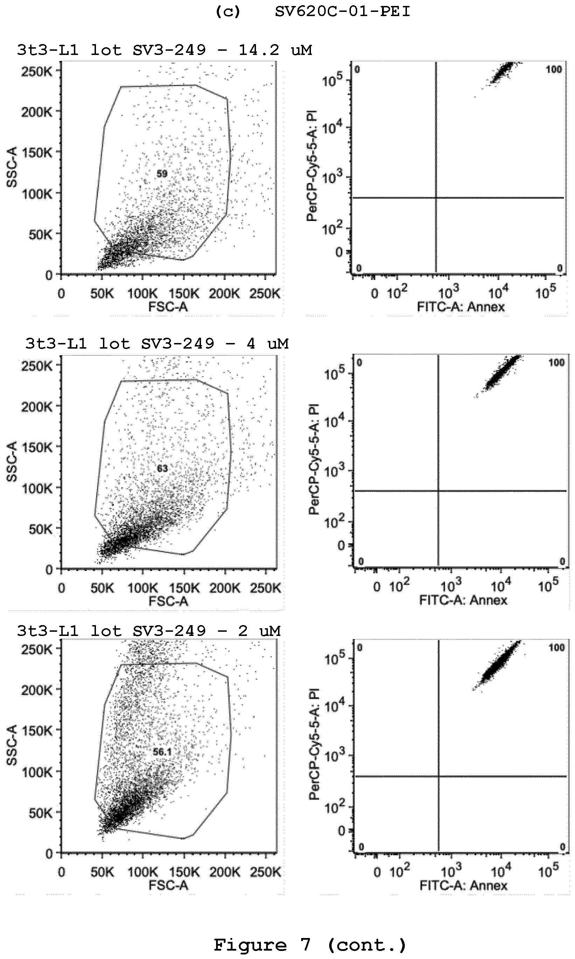

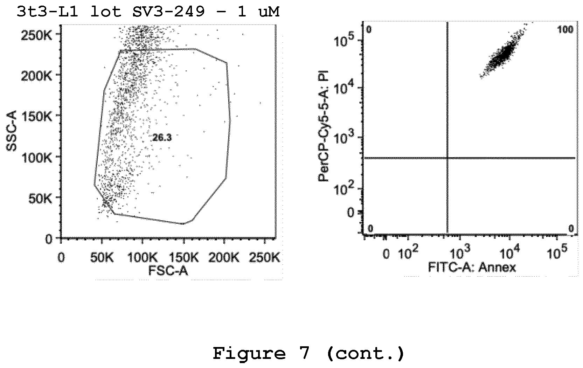

**Figure 7****.** Flow cytometry analysis to evaluate the cytotoxicity of the fluorescent polycationic polymers:
   (a) SV680A-02-WS chitosan,
   (b) SV770Z-01-WS chitosan,
   (c) SV620C-01-PEI.

### DETAILED DESCRIPTION OF THE INVENTION

In the following description, numerous specific details are given to provide a thorough understanding of the embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment.

The headings provided herein are for convenience only and do not interpret the scope or meaning of the embodiments.

The invention relates to fluorescent water-soluble polycationic chitosan polymers and use thereof as markers for imaging glycosamine-containing structures in 3-dimensions.

In one embodiment, the present invention concerns a fluorescent water-soluble polycationic chitosan polymer represented by the general formula (I) and salts thereof: 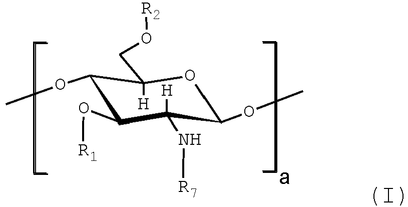 wherein
a is an integer between 220 and 11,200;
R₁ and R₂ are independently selected from the group consisting of CH₃CO and H, provided that at least 25% of the O-positions of the chitosan polymer structure is substituted with CH₃CO;
R₇ is selected from the group consisting of CH₃CO, H and Dye, provided that (i) at least one Dye residue is present in the chitosan polymer structure (wherein the Dye is linked to the polymer structure through a covalent amide bond), (ii) and at least 15% of the N-positions of the chitosan polymer structure is substituted with CH₃CO;
Dye is a polymethine dye, having a general formula (II) : 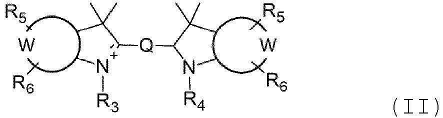
wherein
W represents 4-8 carbon atoms forming a benzocondensed or a naphto-condensed ring;
R₃ and R₄ are independently selected from the group consisting of CH₂CH₃, (CH₂)₃SO³⁻, (CH₂)₃N⁺(CH₃)₃ and salts thereof;
R₅ and R₆ are independently selected from the group consisting of H, SO³⁻ and salts thereof; and
Q is selected from the group consisting of 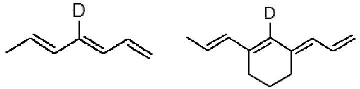
wherein D is selected from the group consisting of

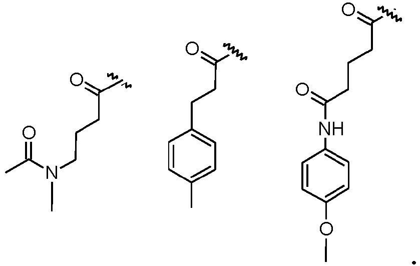

Chitosan is a polysaccharide, composed of β-linked D-glucosamine and *N*-acetyl-D-glucosamine arranged randomly in a linear fashion. Chitosan is readily available by treating chitin with an alkaline compound. Furthermore, chitosan is inexpensive, U.S. Food and Drug Administration (FDA)-approved food additive and has shown no toxicity in biological systems till now [6].

It is well known that chitosan is insoluble in water due to its weak basicity. Therefore, chitosan was acetylated to create its water-soluble variants as reported in Sashiwa H., Kawasaki N., Nakayama A. et. al. [7].

According to the present invention, to realize a water-soluble variant of chitosan, a partial substitution of the amine and hydroxyl groups of the chitosan with water soluble residues has been done, i.e., the substitutions took place only in some of the monomers of the chitosan polymer ('a' structure of formula (I)).

The present inventors finely modulated the degree of acetylation to find an optimal balance between the solubility of the polymer, the labelling of the polymer with the dye for its biological imaging, the amount of positive charge present for the biological tissue staining and selecting the appropriate fluorescent NIR tag to suit the instrumentational/application-based needs of the final user.

In an embodiment, R₁ and R₂ are CH₃CO in at most 50 % of the O-positions of the chitosan polymer structure of formula (I).

In an embodiment, R₁ and R₂ are CH₃CO in a percentage of O-positions of formula (I) between 25% and 50%, preferably between 35% and 45%.

In an embodiment, the fluorescent water-soluble polycationic chitosan polymer of formula (I) has a degree of substitution of R₁ and R₂ with CH₃CO between 0.5 and 1, preferably between 0.8 and 1.

In an embodiment, R₇ is CH₃CO in at most 80 % of the N-positions of formula (I).

In an embodiment, R₇ is CH₃CO in a percentage of N-positions of formula (I) between 15% and 80%.

In an embodiment, R₇ is Dye in at most 50 N-positions of formula (I).

In an embodiment, R₇ is Dye in between 1 and 25 N-positions of formula (I).

In an embodiment, the fluorescent water-soluble polycationic chitosan polymer of formula (I) has a degree of substitution of R₇ with CH₃CO between 0.15 and 0.8, preferably between 0.5 and 0.7.

Dyes belonging to the class of cyanine have already found extensive use in clinical diagnostics; in particular, Indocyanine Green has been used for kidney function tests and fluorescence angiography for more than 30 years [8]. Principally, Cyanine fluorophores that cover the near infrared (NIR) region (700-900 nm) are especially suitable for *in vivo* imaging and therapy due to their improved penetration depth (5-7 mm) as compared with fluorophores that absorb and emit below 600 nm and in the visible wavelength region. In addition, *in vivo* background (or autofluorescence) and scattering from water and chromophores are minimized in the NIR range.

Cyanine 5.5 and cyanine 7 dyes absorb and emit light in the near-infrared region (NIR) (650-900 nm). These dyes are especially suitable for *in vivo* imaging and therapy, since biological tissues absorb poorly in the near-infrared spectral region, thereby allowing NIR dye light to penetrate deeply in such tissues. In addition, these dyes do not get affected from biological tissues' auto-fluorescence in the near-infrared region.

In the current invention, the water-soluble chitosan was conjugated to NIR cyanine dyes, specifically to positively, negatively, and neutrally charged cyanine 5.5 and cyanine 7 dyes. The Cyanine 5.5 and cyanine 7 dyes demonstrate higher chemical and photostability, reproducibility, signal-to-noise ratio, and fluorescent quantum yields as compared to MHI148-PEI. These improved properties also lead to lower dosage for *in vivo* use.

In an embodiment, the Dye residue has an excitation wavelength between 550 nm to 950 nm, preferably 620 nm to 800 nm.

In an embodiment, the polymer has an average molecular weight between 40 kDa and 2,000 kDa, preferably 80 kDa and 500 kDa.

In an embodiment, the polymer has an average molecular weight comprised between 220 and 1120 kDa.

In an embodiment, the Dye residue is selected from formulas (III) and (IV) 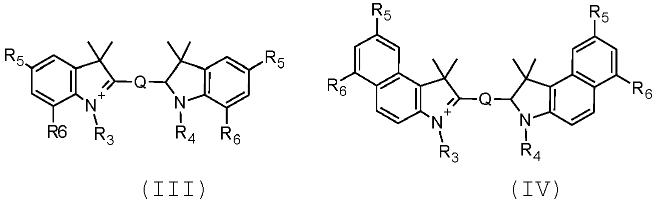 wherein residues R₃-R₆ and Q have the meanings set forth above.

In an embodiment, R₃ and R₄ are independently selected from (CH₂)₃SO³⁻, (CH₂)₃N⁺(CH₃)₃ and salts thereof.

In an embodiment, D is selected from the group consisting of

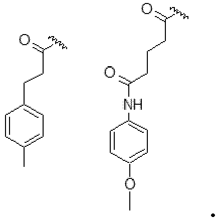

In an embodiment, Q is

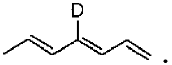

In an embodiment, the Dye residue is a compound of formula (V): 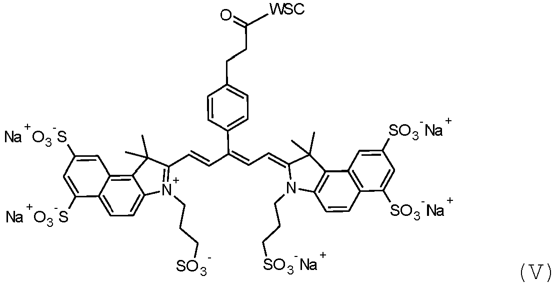 wherein WSC is the water-soluble chitosan polymer that is conjugated to the dye molecule through an amide bond formed in the coupling reaction of carboxyl group of Dye of D 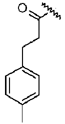 corresponding to the carboxyl group residue having the structure with the NH group present in the N-position of the chitosan polymer of formula (I).

In an embodiment, the Dye residue is a compound of formula (VI) 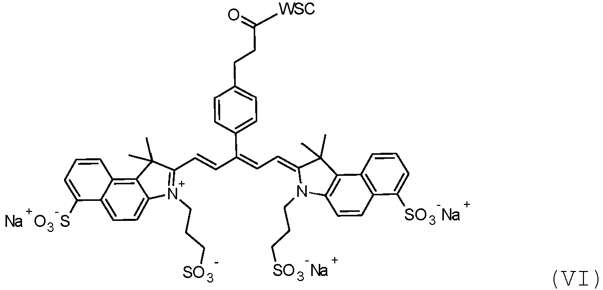 wherein WSC is the water-soluble chitosan polymer that is conjugated to the Dye through an amide bond formed in the coupling reaction of carboxyl group of the Dye corresponding to the carboxyl group of D residue having the structure 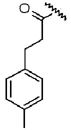 with the NH group present in the *N-*position of the chitosan polymer of formula (I) .

In an embodiment, the Dye residue is a compound of formula (VII) 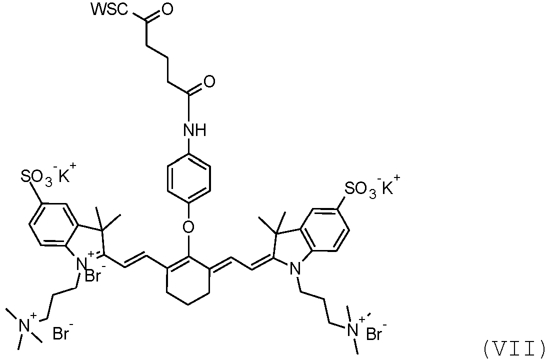 wherein WSC is the water-soluble chitosan polymer that is conjugated to the Dye through an amide bond formed in the coupling reaction of carboxyl group of the Dye corresponding to the carboxyl group of D residue having the structure 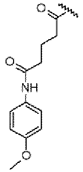 with the NH group present in the *N*-position of the chitosan polymer of formula (I).

In an embodiment, the present invention relates to a fluorescent water-soluble polycationic chitosan polymer of formula (I) for use in an *in vivo* imaging method of a glycosamine-containing structure in a mammal.

In an embodiment, the present invention concerns a fluorescent water-soluble polycationic chitosan polymer of formula (I) for use in a method of imaging a glycosamine-containing structure in a mammal comprising the steps of:
- administering to the mammal a fluorescent water-soluble polycationic chitosan polymer of formula (I);
- detecting the fluorescence emitted by the fluorescent water-soluble polycationic chitosan polymer of formula (I); and
- visualizing the glycosamine-containing structure.

The *in vivo* imaging method allows for visualizing the glycosamine-containing structure for diagnosing a vascular pathology or condition, like for example kidney or lung diseases.

The *in vivo* imaging method allows for assessing the effectiveness of a therapeutic treatment of the glycosamine-containing structure, like for example in case of haemangiomas treatment.

In fact, the imaging method allows visualizing and/or quantifying internal glycosamine-containing structures, preferably inner lumen of blood vessels and/or capillaries, i.e. the vascularization. The term vascularization refers to the density of blood vessels in a tissue or organ and, as will be understood by the skilled person, an increase or a decrease of the vascularization is often a diagnostic finding associated to a pathological state, thereby the disclosed method provides a diagnostic method for determining vascular changes occurring in diseased subjects in comparison to healthy subjects. The vascular staining comprises staining of vessels, peritubular capillaries, glomeruli and pre- or post-glomerular capillaries for providing an indication of pathological conditions, wherein the fluorescent water-soluble polycationic chitosan polymer of formula (I) does not enter the cell but stays in the lumen.

The imaging method herein described also allows for studying the vascularization of organs, preferably kidney.

In an embodiment, the glycosamine-containing structure is selected from blood vessels including vascular networks of capillaries of a mammal, preferably kidney vasculature.

In an embodiment, the glycosamine-containing structure is selected from:
- an endothelial matrix or a cartilage, preferably an endothelial matrix, more preferably an endothelial matrix of a blood vessel, most preferably a basement membrane and/or a glycocalyx of a blood vessel;
- a glomerular filtration barrier matrix, preferably a glomerular basement membrane; and
- a basement membrane and/or a glycocalyx of a capillary, more preferably a basement membrane of pre- or post-glomerular capillaries.

In an embodiment, the polymer of formula (I) is suitable for administration to the mammal and the fluorescent signal emitted from the fluorescent polymer of formula (I) is detected and visualised 3-dimensionally by optical imaging.

In an embodiment, the fluorescent water-soluble polycationic chitosan polymer of formula (I) is suitable for administration to the mammal intravenously or locally.

The imaging method is accomplished in a period that is such to allow the complete binding of the fluorescent water-soluble polycationic chitosan polymer of formula (I) to glycosamine-containing structures in the inner lumen of biological structures. The skilled person understands that the timing depends on specific parameters of the samples, e.g., size, volume and the like.

In an embodiment, the *in vivo* detection of the fluorescence emitted signal from the fluorescent water-soluble polycationic chitosan polymer of formula (I) is performed by means of sensors for fluorescent light, i.e., cameras or little sensors for transcutaneous measurement or intravascular applications.

In an embodiment, the mammal is a laboratory animal selected from a mouse, a rat, a guinea pig, a cat, a dog, a sheep, a goat, a pig, a cow, a horse, and a primate (not a human).

In an embodiment, the mammal is a laboratory mammalian model of kidney disease linked to cardiovascular defects, wherein the disease can be created by causing an appropriate direct or indirect injury and/or by means of a surgical technique, a controlled administration of toxic agents or by means of a transgenesis, wherein transgenesis can be induced by pronuclear injection (additive transgenesis), homologous recombination (targeted transgenesis) or by gene specific silencing of gene function (knock-down) that can be determined by RNA interference (RNAi) or CRISPR/Cas9-based technology [9], [10].

In a further embodiment, the present invention concerns an *in vitro* or *ex vivo* imaging method of glycosamine-containing structures in a biological sample comprising contacting the biological sample with at least one fluorescent water-soluble polycationic chitosan polymer of formula (I) and visualizing the glycosamine-containing structure by means of fluorescence microscopy.

In an embodiment, the detection of the fluorescence emitted signal from the fluorescent water-soluble polycationic chitosan polymer of formula (I) is performed by means of fluorescence microscopy, preferably confocal or light-sheet microscopy, on harvested biological samples, wherein the sample is an isolated organ or a tissue section, preferably a liver, a muscle, a skin, a heart, a kidney, a brain, a lung, more preferably a kidney.

In an embodiment, the imaging method comprises the following steps, preferably in the indicated sequence:
a. Flushing an inner lumen of a biological sample with a washing agent;
b. Contacting said inner lumen with the fluorescent polymer of formula (I);
c. Flushing said inner lumen with a washing agent;
d. Fixing biological structures of the biological sample by flushing said inner lumen with a fixing agent;
e. Optical tissue clearing the biological sample with a clearing agent or alternatively paraffin embedding the biological sample;
f. Optical imaging the biological sample or at least part of it, by means of fluorescence microscopy, preferably confocal and/or light-sheet microscopy.

In a preferred embodiment, the imaging method comprises the following steps:
a. flushing the inner lumen of the biological sample with phosphate-buffer saline (PBS) for at least 5 minutes;
b. contacting the inner lumen with the fluorescent polymer of formula (I) for at least 5 minutes, preferably 10 minutes;
c. flushing the inner lumen with phosphate-buffer saline (PBS) for at least 3 minutes;
d. fixing the biological structures in the biological sample by flushing the inner lumen with a solution containing paraformaldehyde for at least 5 minutes;
e. clearing the tissue(s) of the biological sample with a clearing agent. The term clearing agent refers to a substance that makes the tissue optically transparent for microscopic examination. In case said biological sample is a whole kidney, tissue clearing is preferably performed with ethyl-cinnamate (ECi) following sample-dehydration in ascending concentration of ethanol (50%; 80%;99%) for a total duration of about 4 hours;
f. Optical imaging the biological sample or at least part of it, by means of fluorescence microscopy, preferably confocal and/or light-sheet microscopy. The present invention further concerns a kit comprising at least one fluorescent water-soluble polycationic chitosan polymer of formula (I) and at least one of: a washing agent, a fixing agent, and a clearing agent, preferably in a ready-to-use formulation. Said ready-to-use formulation contains the at least one fluorescent water-soluble polycationic chitosan polymer formula (I) in solid form, which is subsequently dissolved by the user by adding a solvent. The solvent is preferably selected among Milli-Q water and phosphate-buffered saline (PBS).

In an embodiment, the kit contains a washing agent, preferably an isosmotic solution and/or a physiological buffer. Preferably, the washing agent is selected from phosphate-buffered saline (PBS) or Saline/EDTA/Heparin.

In an embodiment, the kit includes a fixing agent for preserving the tissue structures, more preferably a solution of formaldehyde in an isosmotic solution and/or a physiological buffer within a range of from 1% (w/v) to 10% (w/v), preferably 2% to 5% (w/v).

In an embodiment, the kit comprises a clearing agent that does not interfere with the effectiveness of the staining and/or with the detection of the fluorescence of the fluorescent water-soluble polycationic chitosan polymer formula (I), preferably ethyl-cinnamate (ECi).

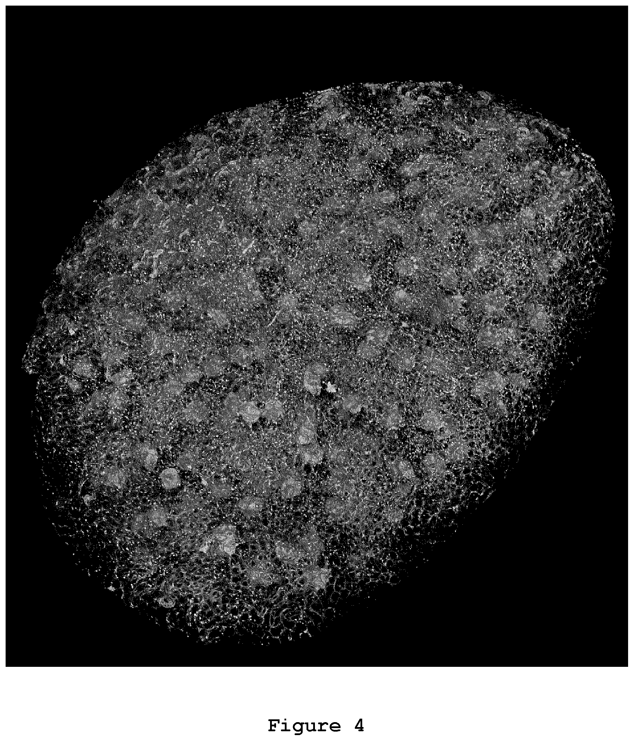

The following examples are intended to illustrate aspects of the present invention and should not be construed as limiting the scope thereof as defined by the claims. They provide a detailed description of the synthesis of the fluorescent water-soluble polycationic chitosan polymer of formula (I), along with the demonstration of its photostability, safety to use in biological testing and biological application as imaging agents (Example 6; Figures 4-6).

### EXAMPLE 1 - Preparation of Dye residue

### a) Preparation of Compound 1 according to Scheme 1.

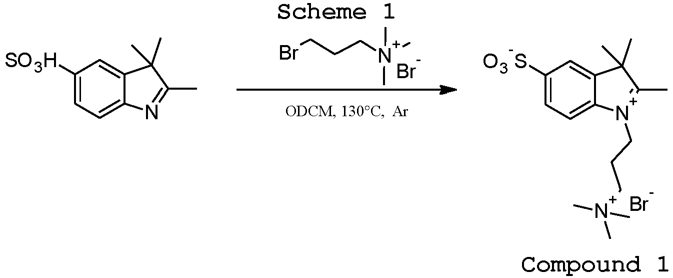

A mixture of 2,3,3-trimethyl-3H-indole-5-sulfonic acid (1.20 g, 5 mmol) and (3-bromopropyl) trimethyl ammonium bromide (1.56 g, 6.0 mmol) in 1,2-dichlorobenzene (16 mL) was heated at 130°C for 72 hours under argon. The mixture was cooled to room temperature and the solvent was decanted. The crude product was washed with dichloromethane, dissolved in acetone and re-precipitated into a large volume of ethyl acetate to obtain a red solid (Compound 1, 1.36 g, yield 80%), which was used in the next step without further purification.

### b) Preparation of Compound 2 according to Scheme 2.

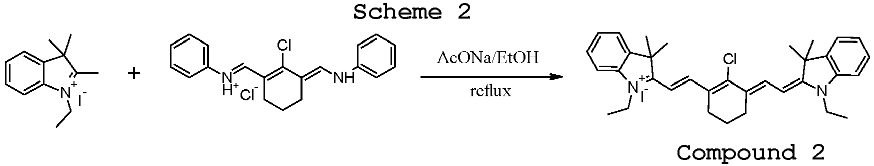

A mixture of 1-ethyl-2,3,3-trimethylindolinium iodide (2 g, 6.34 mmol), Vilsmeier-Haack reagent (1.13 g, 3.17 mmol) and anhydrous sodium acetate (0.93 g, 9.51 mmol) in 20 mL of absolute ethanol was refluxed for 2.5 hours under argon. The reaction mixture was cooled to room temperature, and then concentrated under reduced pressure to yield a brownish green residue. The residue was suspended in dichloromethane, filtered, and dried in vacuum. The crude product was purified by silica gel column chromatography in methanol/dichloromethane = 1/9 to yield a golden-green solid (Compound 2, yield 79.2%). ¹H NMR (300 MHz, CDCl₃): δ 1.46 (t, 6H), 1.72 (s, 12H), 1.99 (m, 2H), 2.77 (t, 4H), 4.24 (q, 4H), 6.25 (d, J=14 Hz, 2H), 7.25(m, 4H), 7.39 (m, 4H), 8.34 (d, J=14 Hz, 2H).

### c) Preparation of Compound 3 according to Scheme 3.

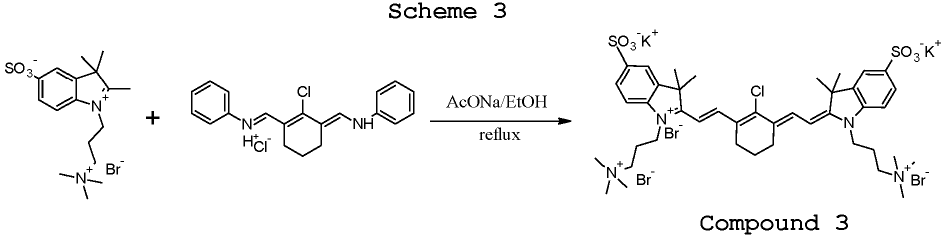

A mixture of bromide salt of compound 1 (0.50 g, 1.48 mmol), Vilsmeier-Haack reagent (0.27 g, 0.73 mmol) and anhydrous sodium acetate (0.25 g, 3.0 mmol) in 10 mL of absolute ethanol was refluxed for 6 hours under argon. The reaction mixture was cooled to room temperature, and then concentrated under reduced pressure to yield a brownish green residue. The crude product was washed with dichloromethane. The residue was suspended in methanol/dichloromethane = 1/4, filtered, and dried in vacuum to yield a golden-green solid (Compound 3, yield 84.9%). ¹H NMR (300 MHz, D₂O): δ 1.72 (s, 12H), 1.88 (m, 2H), 2.18 (m, 4H), 2.74 (m, 4H), 3.08 (s, 18H), 3.49 (m, 4H), 4.18 (m, 4H) m 6.37 (d, J=15 Hz, 2H), 7.45 (d, J=6 Hz, 2H), 7.69 (d, J=6 Hz, 2H) 7.85 (s, 2H), 8.31 (d, J=15 Hz, 2H). LRMS (m/z): calcd: 815.36, found: 815.30.

### d) Preparation of Compound 4 according to Scheme 4.

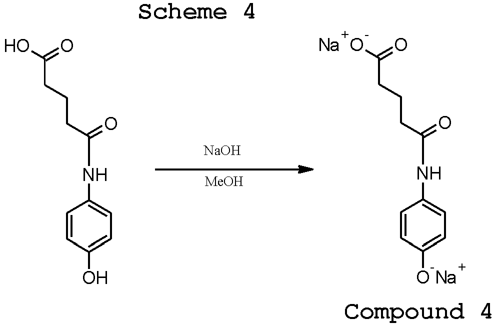

In a 100 mL round-bottom flask, sodium pellets (1.8 g, 45 mmol) were dissolved completely in methanol, and then 4-((4-Hydroxyphenyl)amino)-4-oxobutanoic acid (5 g, 22.5 mmol) was added and stirred altogether at room temperature for 2.5 hours. The reaction was stopped and precipitated in diethyl ether. The precipitate was filtered and dried to obtain a white solid, which was directly used for the next step (Compound 4, 89% yield).

### e) Preparation of SV620C-01 according to Scheme 5.

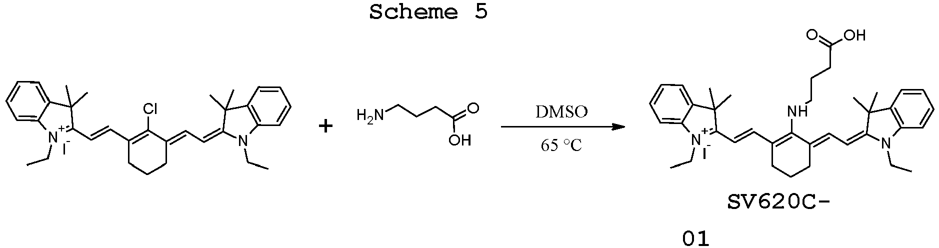

A mixture of compound 2 (800 mg, 1.25 mmol) and 4-aminobutanoic acid (387 mg, 3.75 mmol) in DMSO was heated at 65 °C for 4 hours. The reaction mixture was cooled to room temperature and dissolved in dichloromethane. It was extracted twice in water and once in brine. The organic layer was collected, and dried using sodium sulphate, filtered, and dried overnight. The crude product was purified by silica gel column chromatography in methanol/dichloromethane = 1/9 to yield a golden-blue solid (SV620C-01, yield 42%). ¹H NMR (300 MHz, DMSO) : δ 1.18 (t, 6H), 1.56 (s, 12H), 1.71 (m, 2H), 1.82 (m, 2H), 2.33 (m, 2H), 3.71 (t, 2H), 3.90 (m, 4H), 5.6 (d, J=12 Hz, 2H), 6.98 (m, 4H), 7.23 (t, 2H), 7.35 (d, J=7 Hz, 2H), 7.5 (d, J=12 Hz, 2H).

### f) Preparation of SV770Z-01 according to Scheme 6.

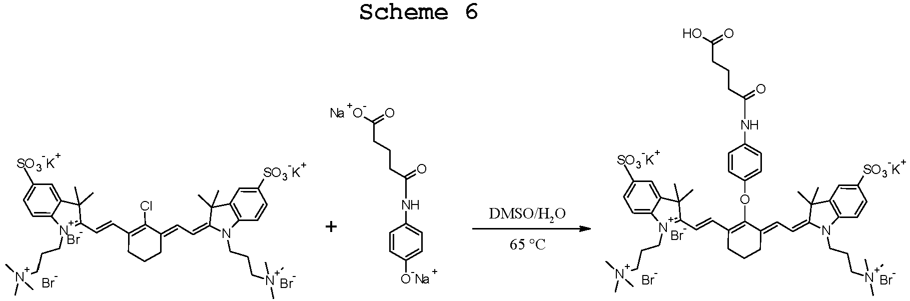

A mixture of compound 3 (500 mg, 0.47 mmol) and compound 4 (502 mg, 1.88 mmol) in DMSO and water (1:1) was heated at 65 °C for 2.5 hours. The reaction mixture was cooled to room temperature and precipitated slowly in ethyl acetate and absolute ethanol (1:1) with 0.1% formic acid. The precipitate was filtered and washed with excess ethanol. The crude product was purified by RP C18 chromatography to yield a golden-green solid compound (SV770Z-01, yield 42%). ¹H NMR (300 MHz, D₂O): δ 1.15 (s, 12H), 1.9 (m, 4H), 2.25 (m, 6H), 2.4 (t, 2H), 2.63 (m, 4H), 3.13 (s, 18H), 3.49 (m, 4H), 4.04 (s, 4H), 6.14 (d, J= 14 Hz, 2H), 6.96 (d, J= 8 Hz, 2H), 7.17 (d, J= 8 Hz, 2H), 7.40 (d, J= 8 Hz, 2H), 7.68 (s, 1H), 7.72 - 7.86 (m, J = 12.0 Hz, 4H).

### g) Preparation of Compound 5 according to Scheme 7.

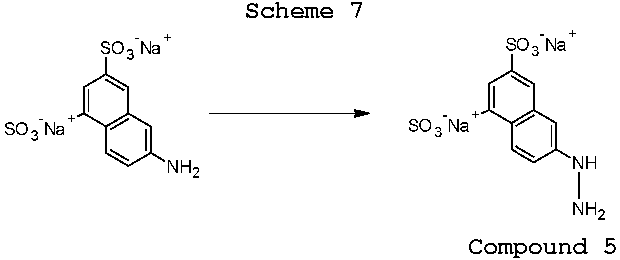

6-amino-1,3-naphthalenedisulfonic acid disodium salt (31.1 g, 89.58 mmol, 1 eq) was added to a 1 L jacketed flask connected to a cryostat at -5°C. Concentrated hydrochloric acid (100 mL) was added very slowly with continuous stirring. Once fully dissolved, a solution of sodium nitrate (9.27 g, 89.58 mmol, 1 eq) in 40 mL water was added very slowly using a dropping funnel over 60 minutes. The reaction was run for 30 mins at 2 °C. Temperature was again brought down to -5 °C and a solution of tin (II) chloride dihydrate (60.62 g, 268.74 mmol, 3 eq) in concentrated hydrochloric acid (60 mL) was added slowly using a dropping funnel over 2 hours. The reaction was allowed to run at 2 °C for 1 hour. The reaction mixture was filtered and washed with excess absolute ethanol. The precipitate was dried under vacuum for 2 days to give compound 5 (yield 50 %). 1H NMR (300 MHz, DMSO-d6): δ 8.72 (d, J = 9.2 Hz, 1H), 8.1 (d, J = 1.2 Hz, 1H), 7.95 (s, 1H), 7.3 (s, 1H), 7.15 (d, J = 9.2 Hz, 1H).

### h) Preparation of Compound 6 according to Scheme 8.

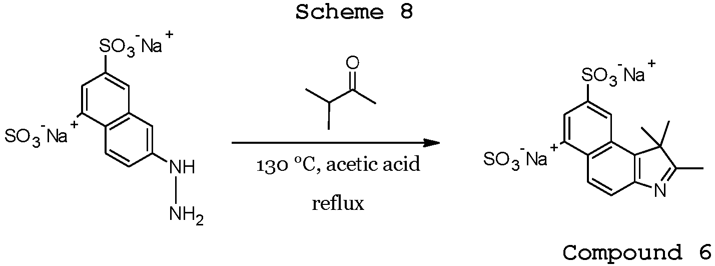

Compound 5 (7 g, 19.3 mmol, 1 eq) was taken in a round bottom flask with 3-methyl-2-butanone (6.125 mL, 57 mmol, 3 eq) and 25 mL of acetic acid. The reaction mixture was refluxed at 130 °C for 5 hours with continuous stirring. The reaction was then cooled down and precipitated into ethyl acetate. The precipitate was filtered over gooch funnel and dried under vacuum to give compound 6 (yield 75%). ¹H NMR (300 MHz, D2O) : δ 8.8 (d, J = 7 Hz, 1H), 8.7 (s, 1H), 8.45 (s, 1H), 7.9 (d, J = 7 Hz, 1H), 2.4 (s, 3H), 1.5 (s, 6H).

### i) Preparation of Compound 7 according to Scheme 9.

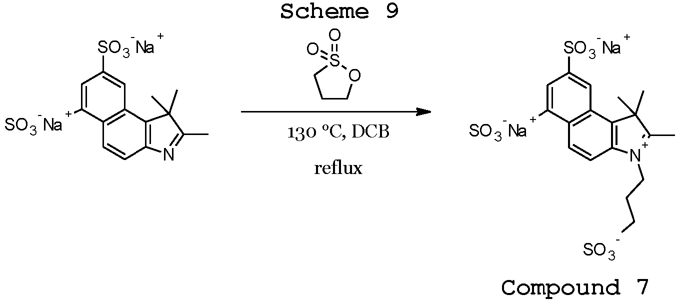

Compound 6 (6.5 g, 14.5 mmol, 1 eq) was taken in a round-bottom flask along with 1,3-propanesultone (2 mL, 21.8 mmol, 1.5 eq) in 1,2-dichlorobenzene (35 mL) and refluxed at 130 °C for 20 hours. The reaction was then cooled down and the solvent was decanted. The precipitate was triturated with 40 mL of ethyl acetate and filtered over gooch funnel. The solid precipitate was further triturated with ethyl acetate (4 X 40 mL) after which it was re-dissolved in hot methanol (100 mL) and precipitated in isopropanol. The precipitate was filtered and washed with isopropanol, diethyl ether and ethyl acetate. The solid was dried under vacuum to give compound 7 (yield 43.4%). Compound 7 was directly used for the next step.

### j) Preparation of Compound 8

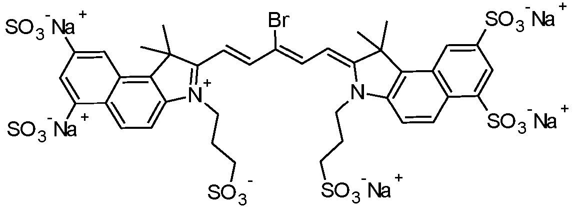

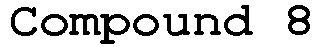

In a 100 mL round-bottom flask, Compound 7 (3.5 g, 6.3 mmol, 2 eq) was stirred along with triethylamine (8.78 mL, 63 mmol, 10 eq) in absolute ethanol (30 mL) at room temperature for 30 mins till it fully dissolved. N-Phenyl-2-bromo-3-(phenylimino)-1-propene-1-amine (1.202 g, 3.15 mmol, 1 eq) and acetic anhydride (10 mL) was added to the flask and the reaction was stirred at 80 °C for 2 hr. The reaction was cooled and precipitated in excess acetone. The precipitate was filtered over gooch funnel and was dried under vacuum. The crude was purified using reverse-phase C18 column chromatography to yield a blue powder (Compound 8, 1.72 g, yield 50 %). 1H-NMR (400 MHz, D₂O): δ 1.81 (s, 12H), 2.16-2.28 (m, 4H), 2.93-3 (m, 4H), 4.36 (t, 4H), 6.33 (d, J = 13.4 Hz, 2H), 7.83 (d, J = 9.4 Hz, 2H), 8.18 (dd, J = 15.2, 7.5 Hz, 4H), 8.64 (d, 2H), 8.77 (d, J = 9.8 Hz, 2H). ε: 240,000 M-1 cm-1. λab: 674 nm, λem: 703 nm [6].

### k) Preparation of SV680A-02

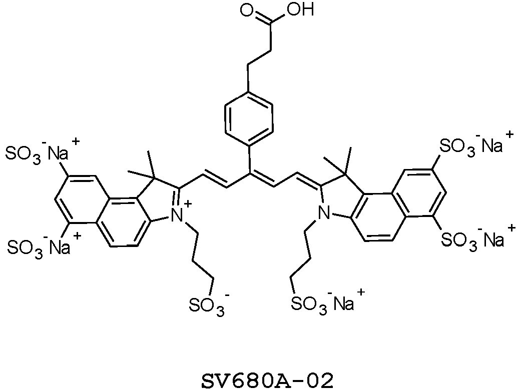

In a 10 mL round-bottom flask under argon atmosphere, Compound 8 (0.09 g, 0.057 mmol, 1 eq) was taken along 4-(2-carboxyethyl) benzene boronic acid (0.033 g, 0.173 mmol, 3 eq) and cesium carbonate (0.037 g, 0.115 mmol, 2 eq) in absolute ethanol and water (50/50) and stirred at room temperature for 30 mins, till it was fully dissolved. Tetrakis(triphenylphosphine)palladium (0) (0.013 g, 0.0115 mmol, 20% by weight) was added to the flask and the temperature was raised to 80 °C. The reaction was stirred for 4 hr and then extracted in ethyl acetate and water. The crude was purified using reverse-phase C18 column chromatography to yield a golden-blue solid (SV680A-02, 0.05 g, yield 80 %). %). 1H-NMR (400 MHz, D₂O): δ 1.95 (s, 12H), 1.99-2.07 (m, 4H), 2.52 (t, J = 7.8 Hz, 2H), 2.66-2.78 (m, J = 7.7 Hz, 4H), 2.93 (t, 2H), 3.92-4.04 (m, 4H), 5.76 (d, J = 14.1 Hz, 2H), 7.26 (d, J = 8.1 Hz, 2H), 7.47 (d, J = 7.8 Hz, 2H), 7.70 (t, J = 9.7 Hz, 2H), 8.20-8.30 (m, 4H), 8.65-8.76 (m, 4H). ε: 240,000 M-1 cm-1. λab: 674 nm, λem: 703 nm [6].

### 1) Preparation of Compound 9

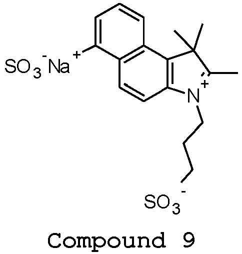

Compound 9 was synthesised using the same procedure as reported in scheme 7, 8 and 9, with the only difference of using 6-Amino-1-naphthalenesulfonic acid as the starting material in Scheme 7.

### m) Preparation of Compound 10 according to Scheme 10

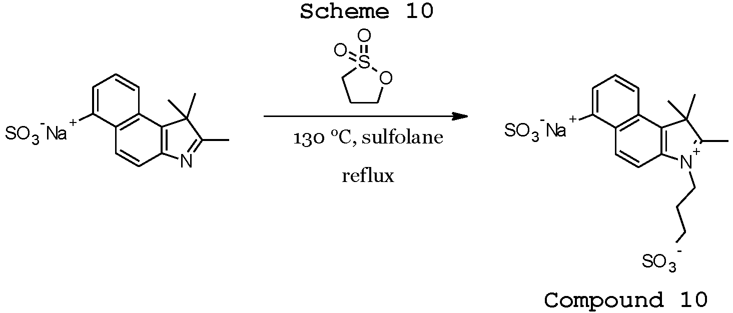

Compound 9 (1.5 g, 4.8 mmol, 1 eq) was taken in a round-bottom flask and dissolved completely in sulfolane (5 mL), to which 1,3-propanesultone (0.631 mL, 7.2 mmol, 1.5 eq) was added. Altogether they were refluxed at 130 °C for 20 hours. The reaction was then cooled down and the solvent was decanted. 5 mL of methanol was added to quench the reaction and dissolve the solid block, which was then precipitated in acetone. The precipitate was filtered over gooch funnel and washed with acetone, ethanol, and diethyl ether. The solid was dried under vacuum to give compound 10 (yield 66.6%). Compound 10 was used for synthesising **SV680A-03** using the same procedure as used for SV680A-02.

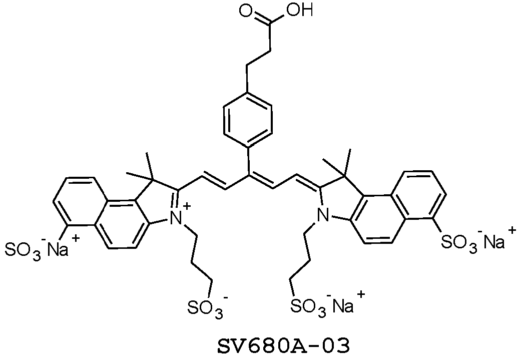

### n) Preparation of SV770C-01 according to Scheme 11

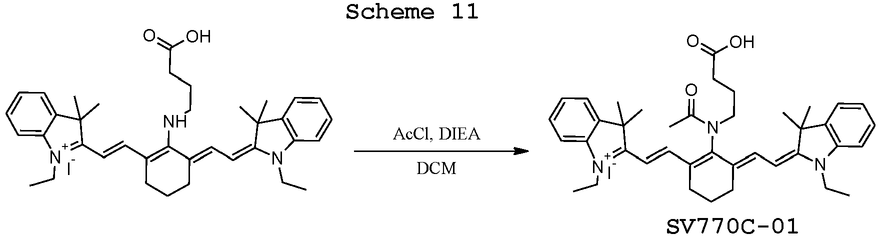

In a 2-necked round-bottom flask, SV620C-01 (50 mg, 0.070 mmol, 1 eq) was dissolved in dichloromethane (12 mL) in an ice bath under argon atmosphere. Acetyl chloride (29.25 µL, 0.375 mmol,5 eq) and *N*, *N-*diisopropylethylamine (240.5 µL, 1.875 mmol, 25 eq) were injected into the reaction flask and stirred for 15 mins. The reaction was quenched in 0.1 N hydrochloric acid solution (20 mL) and then dried off over rotary evaporator. The crude product was purified by silica gel column chromatography in methanol/dichloromethane = 1/9 to yield a golden-green solid (SV770C-01, yield 75%). ¹H NMR (300 MHz, DMSO): δ 1.27 (t, 6H), 1.53 (s, 12H), 2.36 (m, 2H), 1.82 (m, 2H), 2.65 (m, 2H), 3.29 (s, 2H), 3.66 (t, 2H), 4.20 (m, 4H), 6.23 (d, J=12 Hz, 2H), 7.24 (m, 4H), 7.39 (t, 2H), 7.45 (d, J=7 Hz, 2H), 7.55 (d, J=12 Hz, 2H).

### o) Preparation of SV770C-02 according to Scheme 12

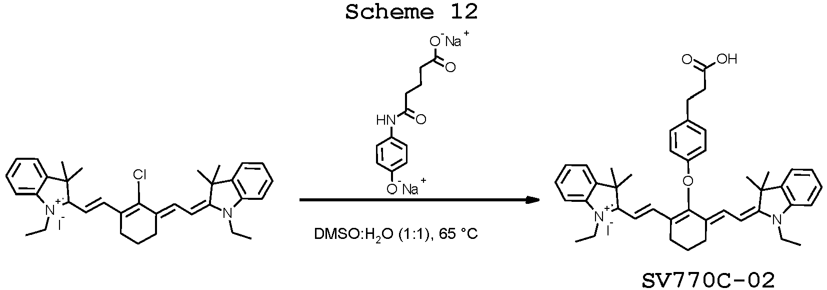

A mixture of compound 2 (912 mg, 1.42 mmol, 1 eq) and compound 4 (1.2 g, 4.2 mmol, 3 eq) in DMSO and water (1:1) was heated at 65 °C for 23 hours. The reaction mixture was cooled to room temperature and acidified with 0.1% formic acid. The reaction was extracted in dichloromethane and water, dried over sodium sulfate and then put on rotary evaporator overnight to give solid crude. The crude product was purified by silica gel chromatography to yield a golden-green solid compound (SV770C-02, yield 42%). ¹H NMR (300 MHz, D₂O): δ 1.15 (s, 12H), 1.9 (m, 4H), 2.25 (m, 6H), 2.4 (t, 2H), 2.63 (m, 4H), 3.13 (s, 18H), 3.49 (m, 4H), 4.04 (s, 4H), 6.14 (d, J= 14 Hz, 2H), 6.96 (d, J= 8 Hz, 2H), 7.17 (d, J= 8 Hz, 2H), 7.40 (d, J= 8 Hz, 2H), 7.68 (s, 1H), 7.72 - 7.86 (m, J = 12.0 Hz, 4H).

### EXAMPLE 2 - Preparation of WS Chitosan

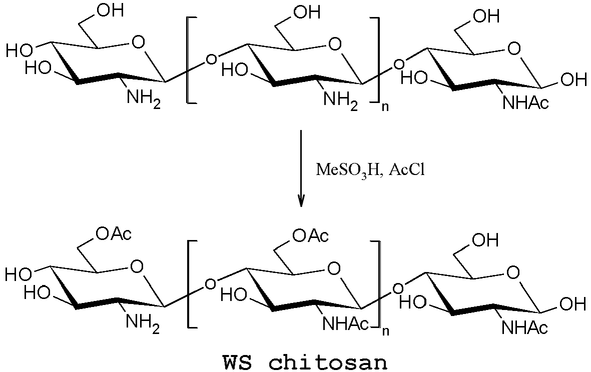

In a 100 mL round-bottom flask, Chitosan (2.2 g, 0.0169 mmol) was stirred in 22 mL of methane sulfonic acid. After 1.5 hours, acetyl chloride was added to this flask, and was stirred altogether for 5 hours. The reaction was stopped by adding 50 g ice to the flask and the reaction mixture was transferred to a dialysis bag (Slide-A-Lyzer^{™} Dialysis Flask, 2K MWCO, 250mL) and dialysed against Milli-Q water. After 36 hours, the content of the dialysis bag was neutralised using sodium bicarbonate solution and continued to dialyse for 2 days. The dialysing medium was changed every 12 hours. After 2 days, the content of the dialysis bag was freeze-dried to give a white solid (*WS Chitosan,* yield 66.4%)

### EXAMPLE 3 - Preparation of fluorescent water-soluble Chitosan polymer of formula (I)

### a) Preparation of SV680A-03-WS Chitosan

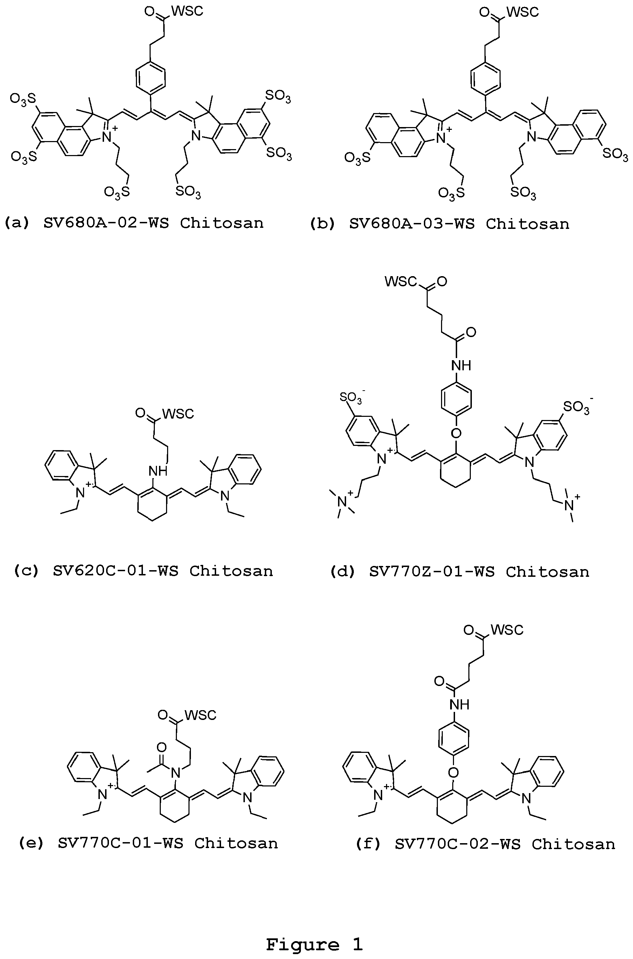

In a 25 mL round-bottom flask, a mixture of SV680A-03 (48 mg, 0.04 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100mg, 0.0009 mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV680A-03-WS Chitosan, 70% yield). The chemical structure of the compound is shown in fig. 1(a).

### b) Preparation of SV680A-02-WS Chitosan

In a 25 mL round-bottom flask, a mixture of SV680A-02 (42.2 mg, 0.036 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100mg, 0.0009\ mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV680A-02-WS Chitosan, 70% yield). The chemical structure of the compound is shown in fig. 1(b).

### c) Preparation of SV620C-01-WS Chitosan

In a 25 mL round-bottom flask, a mixture of SV620C-01 (25 mg, 0.04 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100mg, 0.0009 mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV620C-01-WS Chitosan, 70% yield). The chemical structure of the compound is shown in fig. 1(c).

### d) Preparation of SV770Z-01-WS Chitosan

In a 25 mL round-bottom flask, a mixture of SV770Z-01 (56 mg, 0.04 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100 mg, 0.0009 mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV770Z-01-WS Chitosan, 70% yield). The chemical structure of the compound is shown in fig. 1(d).

### e) Preparation of SV770C-01-WS Chitosan

In a 25 mL round-bottom flask, a mixture of SV770C-01 (25 mg, 0.04 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100mg, 0.0009 mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV770C-01-WS Chitosan, 70% yield). The chemical structure of the compound is shown in fig. 1(e).

### f) Preparation of SV770C-02-WS Chitosan

In a 25 mL round-bottom flask, a mixture of SV770C-02 (56 mg, 0.04 mmol), N-Hydroxysuccinimide (20 mg, 0.18 mmol) and 1-Ethyl-3-(3-dimethylaminopropyl)carbodiimide (28 mg, 0.18 mmol) was stirred in 5 mL anhydrous DMSO at room temperature under Argon for 20 mins. To this reaction mixture, a solution of WS Chitosan (100mg, 0.0009 mmol) in 10 mL anhydrous DMSO was added and stirred altogether for 4.5 hours at room temperature. The reaction was stopped, and the content of the flask was transferred to a dialysis bag (Pur-A-Lyzer^{™} Mega Dialysis Kit, 3.5 MWCO, 3-20 mL). The reaction mix was dialysed against 1X PBS buffer (pH 7.4) for 24 hours with change of the dialysis medium every 12 hours, and later against Milli-Q water for another 12 hours. The resulting solution inside the dialysis bag was lyophilised to obtain a blue powder (SV770C-02-WS Chitosan, 70% yield). The chemical structure of the compound is shown in fig. 1(f).

### EXAMPLE 4 - Photostability studies on the markers

Time dependence photostability evaluation of fluorescent water-soluble chitosan polymers of formula (I) (SV680A-02-WS chitosan, SV680A-03-WS chitosan, SV770Z-01-WS chitosan, SV620C-01-WS chitosan) and the dye SV620C-01 conjugated to PEI were performed under continuous illumination in PBS at 20°C, using Cary 4000 UV-vis spectrophotometer (Agilent) and FS5 spectrofluorometer (Edinburgh Instruments) at University of Parma. Mean emission intensity of the markers was calculated by averaging the experimental values collected from 3 replicative studies. The values were collected at time 0 mins, and after 170 minutes of continuous illumination.

SV620C-01-PEI was compared with SV620C-01-WS chitosan to evaluate the efficiency of WS chitosan as a substitute to PEI, which has been used in the prior art. SV620C-01-WS chitosan was found to be more stable than SV620C-01-PEI, with 40% degradation of the WS chitosan conjugate as compared to 60% degradation of the PEI conjugate. This indicates that WS chitosan offers higher stability to the dye, as compared to PEI, and therefore higher stability in comparison to MHI-148-PEI.

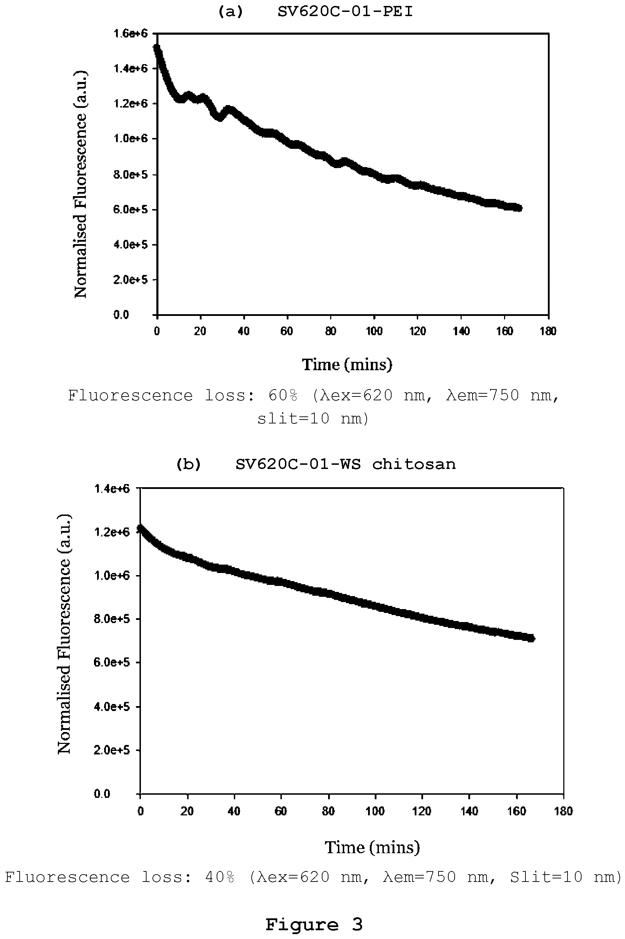

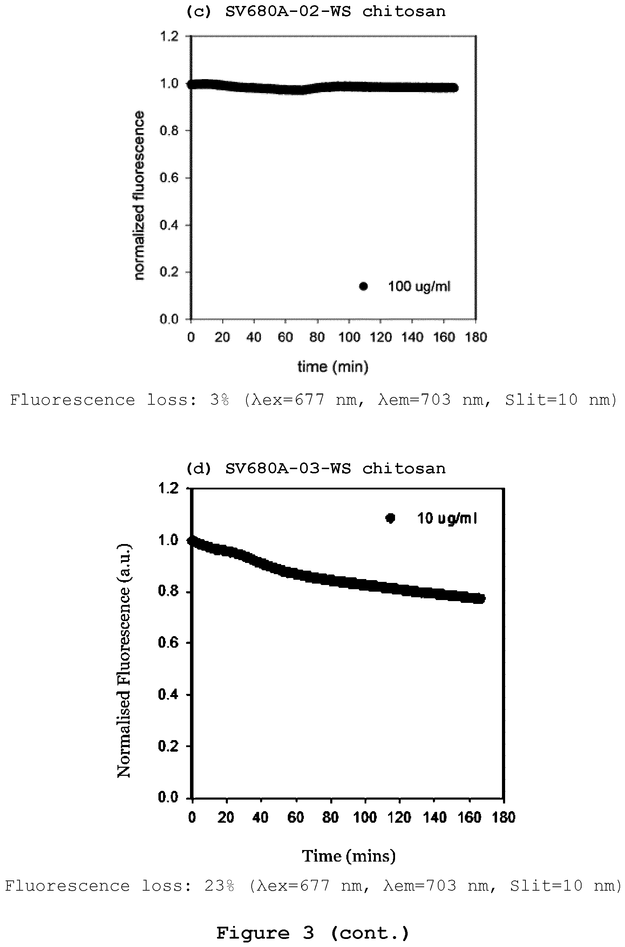

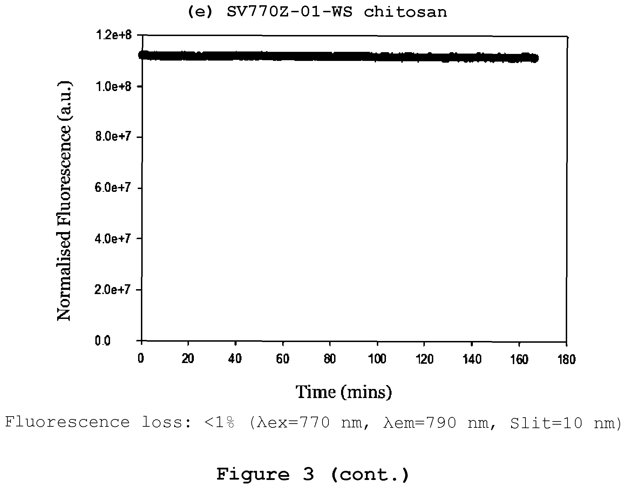

Among the remaining WS chitosan conjugates, SV770Z-01-WS chitosan and SV680A-02-WS chitosan show the highest stability, as seen in Figure 3.

### EXAMPLE 5 - Cytotoxicity assays on the markers

Cytotoxicity assays were performed on the fluorescent water-soluble chitosan polymers of formula (I) (SV680A-02-WS chitosan and SV770Z-01-WS chitosan) and the dye SV620C-01 conjugated to PEI at various concentrations:
- SV680A-02-WS chitosan: 4uM; 2uM; 1uM
- SV770Z-01-WS chitosan: 4uM; 2uM; 1uM
- SV620C-01-PEI: 14.2 µM; 4uM; 2uM; 1uM

The cells were left to incubate with the markers at various concentrations for 48h, after which, they were treated with trypsin and the supernatants and cells were collected.

The samples thus obtained were labelled and analysed following the experimental laboratory procedures, for the evaluation of apoptosis. The test was performed in 6-well plates with seeded 100,000 cells of the line 3T3-L1 (Merck) per well, in a volume of 2 mL of complete DMEM medium (10% FBS, 1% Penicillin / Streptomycin / L-Glutamine). The cells were allowed to adhere to the surface overnight. The next day the fluorescent markers were dissolved in complete DMEM medium, and the treatment was carried out with the cells in 1.5 mL of DMEAM medium with different concentrations of compounds. The cells were left to incubate for 48h. After 48 hours, the supernatants and cells were collected after trypsinization. The samples thus obtained were labelled and analysed following the procedures laboratory tests, for the evaluation of apoptosis. Apoptosis was assessed by flow cytometric analysis.

The percentage of apoptotic cells in the different phases of the apoptotic process, was calculated using the formula: ((test% - control%) _{*} 100 / (100 - control%)).

From the tests carried out it is concluded that SV620C-01-PEI induces 100% cell death at all concentrations tested. The remaining batches, according to our knowledge, do not induce significant apoptosis, although the values indicate a slight increase in specific apoptosis.

### EXAMPLE 6 - Biological performance of the markers

The fluorescent polymers of formula (I) were tested in mice. Organs were harvested and optically cleared following retrograde perfusion.
Animal: C57BL/6 female mice
Marker: SV770Z-01-WS Chitosan and SV680A-02-WS Chitosan
Dosage: 0.5 mg/mL SV770Z-01-WS Chitosan and 2 mg/mL SV680A-02-WS Chitosan

All animal experiments were conducted in accordance with the German Animal Protection Law and approved by the local authority (Regierungspräsidium Nordbaden, Karlsruhe Germany in agreement with EU guideline 2010/63/EU).

Procedure: Mice retrograde perfusion, vascular staining and optical tissue clearing were performed according to the protocol described in Huang et al [1]. Among the collected organs, mouse kidney sections were imaged by confocal microscopy for the 3-dimensional visualization of the vascular structures.

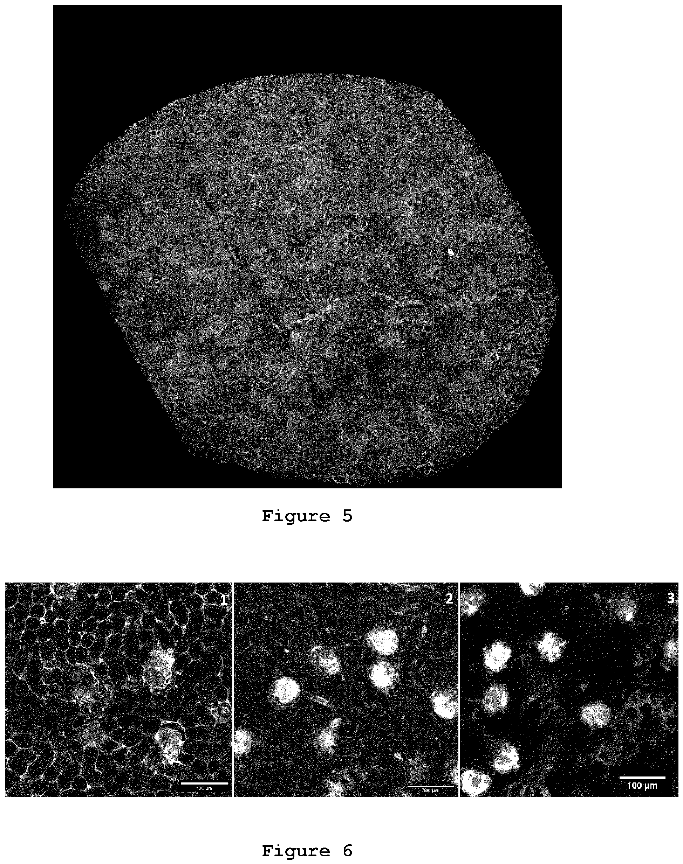

Renal vessels, peritubular capillaries and glomeruli are shown in figure 4 (SV770Z-01-WS Chitosan staining) and figure 5 (SV680A-02-WS Chitosan staining) .

A comparison between the new fluorescent chitosan polymers and the previously disclosed MHI148-PEI is shown in figure 6.

While the visualization of the vascular structures is limited to glomeruli and bigger vessels in kidney samples stained with MHI148-PEI, the use of new fluorescent chitosan polymers of formula (I) allows to display the entire renal vascular network in detail (including peritubular capillaries and post-glomerular capillaries) by administrating a low dosage of these polymers.

### REFERENCES

1. Huang, J., Brenna, C., Khan, A.M. et al. A cationic near infrared fluorescent agent and ethyl-cinnamate tissue clearing protocol for vascular staining and imaging. Sci. Rep. 9, 521 (2019). https://doi.org/10.1038/s41598-018-36741-1.
2. Sivasubramanian M, Hsia Y, Lo L-W. Nanoparticle-facilitated Functional and Molecular Imaging for the Early Detection of Cancer. Front. Mol. Biosci., 1 (2014). https://doi.org/10.3389/fmolb.2014.00015.
3. Gretz, N., Rudolf, R., Weinfurter, S., Brenna, C., Huang, J., Khan, A.M. Means and methods for visualization of tissue structures (2018). WO/2018/100089
4. Exner R.M., Cortezon-Tamarit F., Pascu S.I. Explorations into the Effect of meso-Substituents in Tricarbocyanine Dyes: A Path to Diverse Biomolecular Probes and Materials. Angew. Chem. Int. Ed. 60 (12), 6230-6241 (2021). https://doi.org/10.1002/anie.202008075.
5. Kafil V., Omidi Y. Cytotoxic impacts of linear and branched polyethylenimine nanostructures in a431 cells. Bioimpacts 1(1), 23-30 (2011). https://doi.org/10.5681/bi.2011.004.
6. Qin C., Li H., Xiao Q., Liu Y., Zhu J., Du Y. Water-solubility of chitosan and its antimicrobial activity. Carb Pol. 63(3), 367-374 (2006). https://doi.org/10.1016/j.carbpol.2005.09.023.
7. Sashiwa H., Kawasaki N., Nakayama A., Muraki E., Yamamoto N., Aiba S. Chemical Modification of Chitosan. 14: Synthesis of Water-Soluble Chitosan Derivatives by Simple Acetylation. Biomacromolecules. 3(5), 1126-1128 (2002). https: //doi. org/10. 1021/bm0200480.
8. Muraleedharan S., Tripathy K. Indocyanine Green (ICG) Angiography. [Updated 2022 Mar 28]. In: StatPearls [Internet]. Treasure Island (FL): StatPearls Publishing (2022). https://www.ncbi.nlm.nih.gov/books/NBK580479/
9. Hickman-Davis JM, Davis IC. Transgenic mice. Paediatr Respir Rev. 7(1):49-53(2006). https://doi.org/10.1016/j.prrv.2005.09.005
10. Deutsch M, Günther A, Lerchundi R, Rose CR, Balfanz S, Baumann A. AAV-Mediated CRISPRi and RNAi Based Gene Silencing in Mouse Hippocampal Neurons. Cells. 10(2):324(2021). https://doi.org/10.3390/cells10020324

## Claims

1. A fluorescent water-soluble polycationic chitosan polymer having formula (I) and salts thereof: 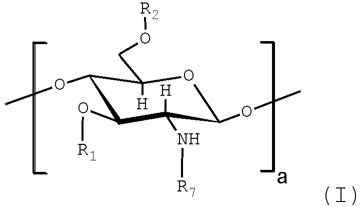 wherein
a is an integer between 220 and 11,200;
R₁ and R₂ are independently selected from the group consisting of CH₃CO and H, provided that at least 25% of the O-positions of the chitosan polymer structure is substituted with CH₃CO;
R₇ is selected from the group consisting of CH₃CO, H and Dye, provided that (i) at least one Dye residue is present in the chitosan polymer structure, (ii) and at least 15% of the N-positions of the chitosan polymer structure is substituted with CH₃CO;
Dye is a polymethine dye, having a general formula (II) : 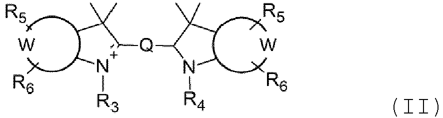
wherein
W represents 4-8 carbon atoms forming a benzocondensed or a naphto-condensed ring;
R₃ and R₄ are independently selected from the group consisting of CH₂CH₃, (CH₂)₃SO³⁻, (CH₂)₃N⁺(CH₃)₃;
R₅ and R₆ are independently selected from the group consisting of H, SO³⁻;
Q is selected from the group consisting of 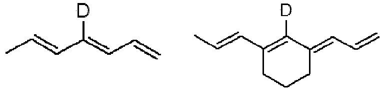
wherein D is selected from the group consisting of

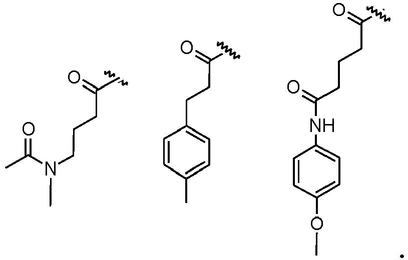

2. The fluorescent water-soluble polycationic chitosan polymer according to claim 1, wherein R₁ and R₂ are CH₃CO in at most 50% of the O-positions of formula (I).

3. The fluorescent water-soluble polycationic chitosan polymer according to any one of the preceding claims, R₇ is CH₃CO in at most 80 % of the N-positions of formula (I).

4. The fluorescent water-soluble polycationic chitosan polymer according to any one of the preceding claims, wherein R₇ is Dye in at most 50 N-positions of formula (I).

5. The fluorescent water-soluble polycationic chitosan polymer according to any one of the preceding claims, wherein the Dye residue has an excitation wavelength ranging from 550 nm to 950 nm.

6. The fluorescent water-soluble polycationic chitosan polymer according to any one of the preceding claims, wherein the fluorescent polymer of formula (I) has an average molecular weight between 40 kDa and 2,000 kDa.

7. The fluorescent water-soluble polycationic chitosan polymer according to any one of the preceding claims, wherein a is comprised between 220 and 1100.

8. The fluorescent water-soluble polycationic chitosan polymer according to any one of the preceding claims, wherein the Dye residue is selected from formulas (III) and (IV) 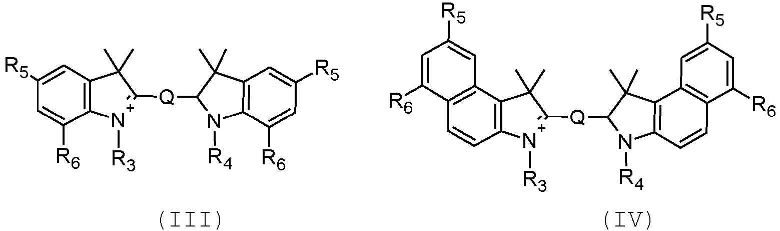 wherein residues R₃-R₆ and Q have the meanings set forth in claim 1.

9. The fluorescent water-soluble polycationic chitosan polymer according to any one of the preceding claims, wherein R₃ and R₄ are independently selected from (CH₂)₃SO³⁻, (CH₂)₃N⁺(CH₃)₃.

10. The fluorescent water-soluble polycationic chitosan polymer according to any one of the preceding claims, wherein Q is

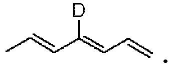

11. The fluorescent water-soluble polycationic chitosan polymer according to any one of the preceding claims, wherein D is selected from the group consisting of

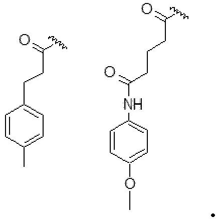

12. A composition comprising a fluorescent, water-soluble polycationic chitosan polymer of formula (I) according to any one of claims 1 to 11 and a carrier or vehicle.

13. A fluorescent water-soluble polycationic chitosan polymer of formula (I) according to any one of claims 1 to 11 for use in an imaging method of a glycosamine-containing structure in a mammal.

14. An *in vitro* or *ex vivo* imaging method of a glycosamine-containing structure in a biological sample comprising:
- contacting the biological sample with at least one fluorescent water-soluble polycationic chitosan polymer of formula (I) according to any one of claims 1 to 11, and
- visualizing the glycosamine-containing structure by means of fluorescence microscopy.

15. A kit comprising at least one fluorescent water-soluble polycationic chitosan polymer of formula (I) according to any one of claims 1 to 11, and at least one of: a washing agent, a fixing agent, and a clearing agent.

## Patentansprüche

1. Fluoreszierendes wasserlösliches polykationisches Chitosanpolymer mit der Formel (I) und Salze davon: 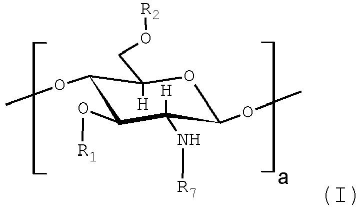 wobei
a eine ganze Zahl zwischen 220 und 11.200 ist;
R₁ und R₂ unabhängig voneinander ausgewählt sind aus der Gruppe bestehend aus CH₃CO und H, vorausgesetzt, dass mindestens 25 % der O-Positionen der Chitosanpolymerstruktur mit CH₃CO substituiert sind;
R₇ ausgewählt ist aus der Gruppe bestehend aus CH₃CO, H und Farbstoff, vorausgesetzt, dass (i) mindestens ein Farbstoffrest in der Chitosanpolymerstruktur vorhanden ist, (ii) und mindestens 15 % der N-Positionen der Chitosanpolymerstruktur mit CH₃CO substituiert sind;
Farbstoff ein Polymethinfarbstoff ist, mit der allgemeinen Formel (II): 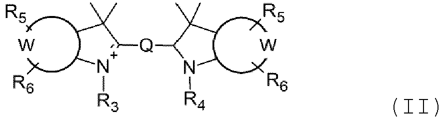
wobei
W für 4-8 Kohlenstoffatome steht, die einen benzokondensierten oder einen naphtho-kondensierten Ring bilden;
R₃ und R₄ unabhängig voneinander ausgewählt sind aus der Gruppe bestehend aus CH₂CH₃, (CH₂)₃SO³⁻, (CH₂)₃N⁺(CH₃)₃;
R₅ und R₆ unabhängig voneinander ausgewählt sind aus der Gruppe bestehend aus H, SO³⁻;
Q ausgewählt ist aus der Gruppe bestehend aus: 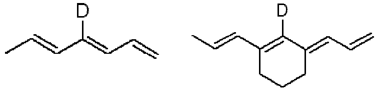
wobei D ausgewählt ist aus der Gruppe bestehend aus

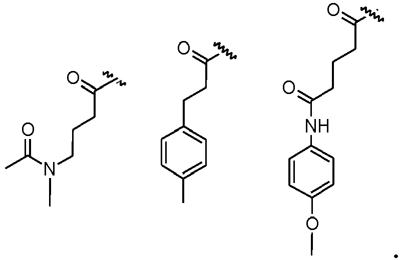

2. Fluoreszierendes wasserlösliches polykationisches Chitosanpolymer nach Anspruch 1, wobei R₁ und R₂ in höchstens 50 % der O-Positionen der Formel (I) CH₃CO sind.

3. Fluoreszierendes, wasserlösliches, polykationisches Chitosanpolymer nach einem der vorhergehenden Ansprüche, wobei R₇ in höchstens 80 % der *N*-Positionen der Formel (I) CH₃CO ist.

4. Fluoreszierendes, wasserlösliches, polykationisches Chitosanpolymer nach einem der vorhergehenden Ansprüche, wobei R₇ in höchstens 50 N-Positionen der Formel (I) Farbstoff ist.

5. Fluoreszierendes wasserlösliches polycationisches Chitosanpolymer nach einem der vorhergehenden Ansprüche, wobei der Farbstoffrest eine Anregungswellenlänge im Bereich von 550 nm bis 950 nm aufweist.

6. Fluoreszierendes wasserlösliches polykationisches Chitosanpolymer nach einem der vorhergehenden angehenden Ansprüche, wobei das fluoreszierende Polymer der Formel (I) ein durchschnittliches Molekulargewicht zwischen 40 kDa und 2.000 kDa aufweist.

7. Fluoreszierendes wasserlösliches polykationisches Chitosanpolymer nach einem der vorhergehenden Ansprüche, wobei a zwischen 220 und 1100 liegt.

8. Fluoreszierendes wasserlösliches polycationisches Chitosanpolymer nach einem der vorhergehenden Ansprüche, wobei der Farbstoffrest aus den folgenden Formeln (III) und (IV) ausgewählt ist: 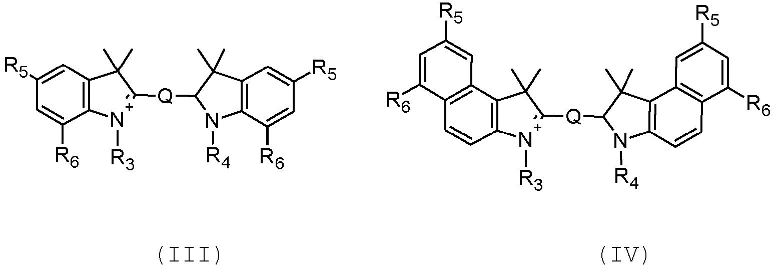 wobei die Reste R₃-R₆ und Q die in Anspruch 1 angegebene Bedeutung haben.

9. Fluoreszierendes wasserlösliches polycationisches Chitosanpolymer nach einem der vorhergehenden Ansprüche, wobei R₃ und R₄ unabhängig voneinander ausgewählt sind aus (CH₂)₃SO³⁻, (CH₂)₃N⁺(CH₃)₃.

10. Fluoreszierendes, wasserlösliches, polykationisches Chitosanpolymer nach einem der vorhergehenden Ansprüche, wobei Q 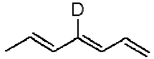 ist.

11. Fluoreszierendes, wasserlösliches, polykationisches Chitosanpolymer nach einem der vorhergehenden Ansprüche, wobei D ausgewählt ist aus der Gruppe bestehend aus:

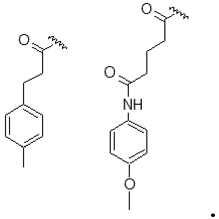

12. Zusammensetzung, umfassend ein fluoreszierendes, wasserlösliches polykationisches Chitosanpolymer der Formel (I) nach einem der Ansprüche 1 bis 11 und einen Träger oder ein Vehikel.

13. Fluoreszierendes, wasserlösliches, polykationisches Chitosanpolymer der Formel (I) nach einem der Ansprüche 1 bis 11 zur Verwendung in einem Verfahren zur Abbildung einer glycosaminhaltigen Struktur in einem Säugetier.

14. *In-vitro-* oder *Ex*-*vivo*-Verfahren zur Abbildung einer glycosaminhaltigen Struktur in einer biologischen Probe, umfassend:
- Inkontaktbringen der biologischen Probe mit mindestens einem fluoreszierenden wasserlöslichen polykationischen Chitosanpolymer der Formel (I) nach einem der Ansprüche 1 bis 11 und
- Sichtbarmachen der glycosaminhaltigen Struktur mittels Fluoreszenzmikroskopie.

15. Kit, umfassend mindestens ein fluoreszierendes, wasserlösliches, polykationisches Chitosanpolymer der Formel (I) nach einem der Ansprüche 1 bis 11 und mindestens eines aus: einem Waschmittel, einem Fixiermittel und einem Klärmittel.

## Revendications

1. Polymère de chitosane polycationique soluble dans l'eau fluorescent de formule (I), et ses sels : 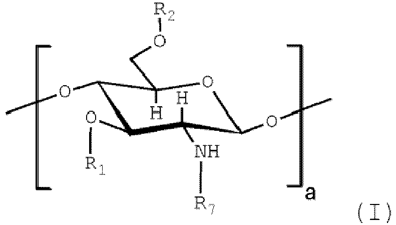 dans lesquels
a est un entier compris entre 220 et 11 200 ;
R₁ et R₂ sont indépendamment choisis dans le groupe constitué par CH₃CO et H, sous réserve qu'au moins 25 % des positions *O* de la structure de polymère de chitosane soient substitués par CH₃CO ;
R₇ est choisi dans le groupe constitué par CH₃CO, H et Dye, sous réserve que (i) au moins un résidu Dye soit présent dans la structure de polymère de chitosane, et (ii) au moins 15 % des positions *N* de la structure de polymère de chitosane soient substitués par CH₃CO ;
Dye est un colorant polyméthine, de formule générale (II) : 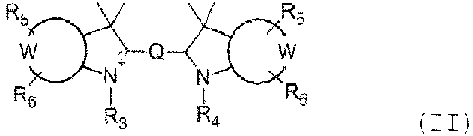
dans lequel
W représente 4 à 8 atomes de carbone formant un cycle benzo-condensé ou naphto-condensé ;
R₃ et R₄ sont indépendamment choisis dans le groupe constitué par CH₂CH₃, (CH₂)₃SO³⁻, (CH₂)₃N⁺(CH₃)₃ ;
R₅ et R₆ sont indépendamment choisis dans le groupe constitué par H, SO³⁻ ;
Q est choisi dans le groupe constitué par 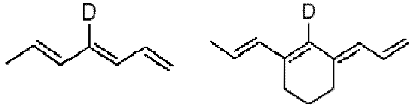
dans lequel D est choisi dans le groupe constitué par

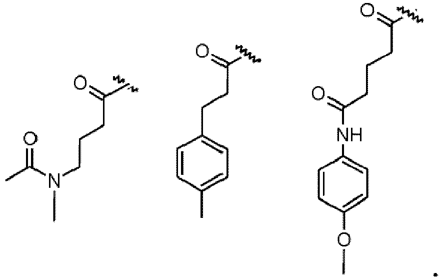

2. Polymère de chitosane polycationique soluble dans l'eau fluorescent selon la revendication 1, dans lequel R₁ et R₂ sont CH₃CO dans au plus 50 % des positions *O* de la formule (I).

3. Polymère de chitosane polycationique soluble dans l'eau fluorescent selon l'une quelconque des revendications précédentes, dans lequel R₇ est CH₃CO dans au plus 80 % des positions *N* de la formule (I).

4. Polymère de chitosane polycationique soluble dans l'eau fluorescent selon l'une quelconque des revendications précédentes, dans lequel R₇ est Dye dans au plus 50 positions *N* de la formule (I).

5. Polymère de chitosane polycationique soluble dans l'eau fluorescent selon l'une quelconque des revendications précédentes, dans lequel le résidu Dye a une longueur d'onde d'excitation située dans la plage allant de 550 nm à 950 nm.

6. Polymère de chitosane polycationique soluble dans l'eau fluorescent selon l'une quelconque des revendications précédentes, dans lequel le polymère fluorescent de formule (I) a une masse moléculaire moyenne comprise entre 40 kDa et 2 000 kDa.

7. Polymère de chitosane polycationique soluble dans l'eau fluorescent selon l'une quelconque des revendications précédentes, dans lequel a est compris entre 220 et 1 100.

8. Polymère de chitosane polycationique soluble dans l'eau fluorescent selon l'une quelconque des revendications précédentes, dans lequel le résidu Dye est choisi parmi les formules (III) et (IV) 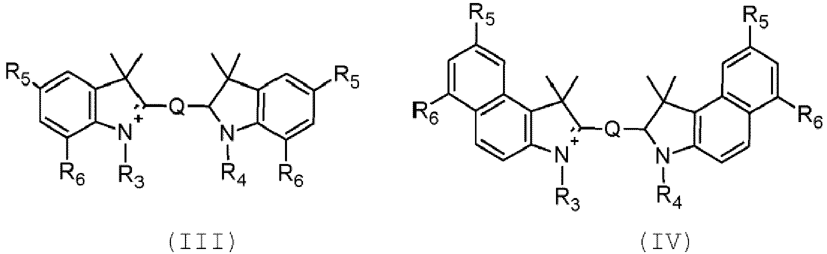 dans lequel les résidus R₃-R₆ et Q ont les significations indiquées dans la revendication 1.

9. Polymère de chitosane polycationique soluble dans l'eau fluorescent selon l'une quelconque des revendications précédentes, dans lequel R₃ et R₄ sont indépendamment choisis parmi (CH₂)₃SO³⁻, (CH₂)₃N⁺(CH₃)₃.

10. Polymère de chitosane polycationique soluble dans l'eau fluorescent selon l'une quelconque des revendications précédentes, dans lequel Q est

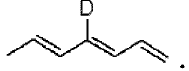

11. Polymère de chitosane polycationique soluble dans l'eau fluorescent selon l'une quelconque des revendications précédentes, dans lequel D est choisi dans le groupe constitué par

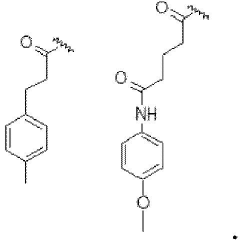

12. Composition comprenant un polymère de chitosane polycationique soluble dans l'eau fluorescent de formule (I) selon l'une quelconque des revendications 1 à 11 et un support ou véhicule.

13. Polymère de chitosane polycationique soluble dans l'eau fluorescent selon l'une quelconque des revendications 1 à 11 pour une utilisation dans une méthode d'imagerie d'une structure contenant une glycosamine chez un mammifère.

14. Méthode d'imagerie *in vitro* ou *ex vivo* d'une structure contenant une glycosamine dans un échantillon biologique comprenant :
- la mise en contact de l'échantillon biologique avec au moins un polymère de chitosane polycationique soluble dans l'eau fluorescent de formule (I) selon l'une quelconque des revendications 1 à 11, et
- la visualisation de la structure contenant une glycosamine au moyen d'une microscopie de fluorescence.

15. Trousse comprenant au moins un polymère de chitosane polycationique soluble dans l'eau fluorescent de formule (I) selon l'une quelconque des revendications 1 à 11, et au moins l'un parmi : un agent de lavage, un agent de fixation, et un agent de transparisation.
